Europäisches Patentamt

(19) European Patent Office

Office européen des brevets

(11) Publication number: **0 231 010**
A2

# EUROPEAN PATENT APPLICATION

(21) Application number: 87101071.6

(22) Date of filing: 27.01.87

(51) Int. Cl.³: **G 01 N 33/543**
C 12 Q 1/68, B 01 D 57/00
G 01 N 33/535, C 12 Q 1/00

(30) Priority: 27.01.86 US 822749
23.01.87 US 6523

(43) Date of publication of application:
05.08.87 Bulletin 87/32

(84) Designated Contracting States:
AT BE CH DE ES FR GB GR IT LI LU NL SE

(71) Applicant: INCSTAR Corporation
P.O. Box 285 1951 Northwestern Avenue
Stillwater Minnesota 55082(US)

(72) Inventor: Halsey, John F.
9001 Maple
Overland Park Kansas 66207(US)

(72) Inventor: Puckett, Larry D.
Box 131 340 Elm Street
Marine-On-St. Croix, MN 55047(US)

(72) Inventor: Halsey, Sandra M.
9001 Maple
Overland Park Kansas 66207(US)

(72) Inventor: Santore, David T.
13901 Tomahawk Lane
Afton Minnesota 55001(US)

(72) Inventor: Colwell, Mark S.
6924 West 77th Terace
Overland Park Kansas 66207(US)

(72) Inventor: McClung, James E.
516 Hud Street
Somerset Wisconsin 54025(US)

(72) Inventor: Heinzerling, Rollin H.
7420 Longview Circle
Chanhassen Minnesota 55317(US)

(74) Representative: Strehl, Schübel-Hopf, Groening, Schulz
Widenmayerstrasse 17 Postfach 22 03 45
D-8000 München 22(DE)

(54) A method of solid phase enzyme immunoassay and nucleic acid hybridization assay and dip-stick design and stabilized chromogenic substrate.

(57) Improved solid phase enzyme immunoassay and solid phase nucleic acid hybridization assay wherein a chromogenic material, capable of changing from a first color state to a second color state in correlation to the amount of enzyme bound to the solid phase, binds to the solid phase. The solid phase is thus analyzed for the second color state. Also, an improved dip-stick design wherein the reactive membrane is thin and planar with both sides of the membrane exposed for contact with reagents. Still further, a stabilized chromogenic solution wherein the chromogen contains a benzidine moiety and is stabilized with a complementary redox reagent.

0231010

## A METHOD OF SOLID PHASE ENZYME IMMUNOASSAY AND NUCLEIC ACID HYBRIDIZATION ASSAY AND DIP-STICK DESIGN AND STABILIZED CHROMOGENIC SUBSTRATE

### BACKGROUND OF THE INVENTION

1.  Field of the Invention.

This invention relates to a method of solid phase enzyme immunoassay and solid phase enzyme nucleic acid hybridization assay in which the chromogenic material, upon changing color in response to the presence of the enzyme on the solid phase, binds to the solid phase. Thus, the solid phase is analyzed for the presence of color change. This invention also relates to a dip-stick design useful in practicing the foregoing method. This invention still further relates to a stabilized chromogenic substrate useful in practicing the foregoing method.

2.  Description of the Prior Art.

Incorporating a solid phase into the method of existing assays has made the requisite separation steps easier to perform. For example, by incorporating a solid phase in a fluorescence immunoassay, bound and unbound fluorescent label material may be easily separated without the need for cumbersome double antibody precipitation. This separation is accomplished by merely separating the solid phase containing bound fluorescent label from the liquid phase containing unbound fluorescent label. This is of great advantage in automated solid

- 2 -

phase fluorescence immunoassays where microfiltration is used to separate the solid from liquid phase. Moreover, the solid phase may be analyzed for the presence of the fluorescent label. The fluorescence of the residual solid phase located on the microfiltration membrane is analyzed for fluorescence.

In contrast, when using enzyme labels the solid phase containing the bound enzyme label must be contacted with an enzyme substrate containing a chromogen and usually a redox reagent. Reaction of the enzyme with the redox reagent causes a color change in the chromogen. Because the chromogen is dissolved in solution, the color appears in the solution rather than on the solid phase. Thus, there is a need for an enzyme assay method in which the color change appears on the solid phase in order to derive benefits generally attributable to radioiodine and especially fluorescence solid phase immunoassays. Thus, there is a need for a solid phase enzyme assay where the color change appears on the solid phase. This would enable enzyme assays to be useful in automated clinical diagnosis where such assays often use a particulate solid phase and in dip-sticks for use in the physician's office.

There is a further need for a redesign of the dip-stick embodiment of the solid phase in order to take full advantage of an assay method where the color change occurs on the solid phase. There is still a further need for a stabilized chromogen which would eliminate the need for preparing an unstable chromogen substrate immediately before performing the enzyme assay as is common in the prior art.

0231010

## SUMMARY OF THE INVENTION

In accordance with the present invention, solid phase enzyme immunoassays and solid phase enzyme nucleic acid hybridization assays are disclosed which incorporate a chromogenic material capable of changing from a first color state to a second color state in correlation to the amount of enzyme label bound to the solid phase. The present invention provides that when in the second color state the chromogen substantially binds to the solid phase. Thus, by analyzing the solid phase for the presence of the second color state, the amount of enzyme label bound to the solid phase may be determined. This invention will be useful for any enzyme immunoassay or enzyme nucleic acid hybridization assay in which the amount of enzyme bound to the solid phase correlates to the presence of analyte in the sample to be assayed.

The solid phase, i.e. solid surface, of the present invention must be available for contact with the chromogenic material. This may be accomplished in the case where the solid surface is a membrane in many forms, including a membrane contained in a dip-stick, a membrane contained in a microtiter well, and a treated or coated surface of a test tube.

In the case of an immunoassay, the invention would be useful for assay configurations, for example, in which the solid phase, i.e. solid surface, has attached to it an immunoreactant specific for the analyte and where the enzyme label is attached to a material which either selectively binds with the analyte or selectively binds with the immunoreactant. One example of an assay configuration where the enzyme-labeled material selectively binds with the analyte is a sandwich assay where the analyte is sandwiched between the immunoreactant attached to the solid surface and the enzyme-labeled material.

Examples of assay configurations where the enzyme-labeled material specifically binds with the immunoreactant include competitive assay configurations, sequential saturation assay configurations, as well as competitive displacement assay configurations.

In the case of nucleic acid hybridization assays, the invention would be useful for assay configurations, for example, in which the solid surface has attached to it a single-stranded first fragment of nucleic acid having a nucleotide sequence of at least 15 base pairs and being capable of hybridizing with the single-stranded analyte nucleic acid present in a liquid sample. The enzyme label is attached to a single-stranded second fragment of nucleic acid also having a nucleotide sequence of at least 15 base pairs where the second fragment of nucleic acid either selectively hybridizes with the analyte nucleic acid or selectively hybridizes with the first fragment of nucleic acid. As in the case of the immunoassay, the presence of analyte nucleic acid in a liquid sample correlates to the amount of enzyme-labeled second fragment of nucleic acid bound by hybridization to the solid surface following reactions of the foregoing. Also, as in the case of the immunoassay, a sandwich configuration may be used where the analyte is sandwiched between the labeled second fragment of nucleic acid and the first fragment attached to the solid phase. The competitive configuration, as well as sequential saturation and competitive displacement configurations, may be used where the labeled second fragment of nucleic acid hybridizes with the first fragment of nucleic acid attached to the solid surface.

For immunoassays, the order in which the immunoreactant attached to the solid surface, the liquid sample, and the enzyme-labeled material are contacted and the incubation conditions of such contact depend upon the assay configuration chosen. The foregoing three reactants may be contacted simultaneously or sequentially in known ways. Analogously, the contacting of the first fragment of nucleic acid attached to the solid surface, the liquid sample, and the enzyme-labeled second fragment of nucleic acid may be contacted simultaneously or sequentially and under appropriate incubation conditions according to the chosen assay configuration.

In the case of an immunoassay, the immunoreactant being "specific" for the analyte means that upon immunoreaction the analyte and immunoreactant will be bound. This is illustrated, for example, by (1) the immunoreactant being an antigen while the analyte is a monoclonal or polyclonal antibody specific for that antigen; or (2) the immunoreactant being a monoclonal or polyclonal antibody specific for an analyte which is an antigen or a second antibody. The enzyme-labeled material which "selectively binds" with either the immunoreactant or the analyte may do so by virtue of immunoreaction (involving polyclonal or monoclonal antibodies or ligands) or chemical reaction or complementation or other known binding mechanisms. In the case of the nucleic acid hybridization assay, the nucleic acid may be any type of DNA or RNA, including naturally-derived or synthetically-produced fragments.

In the case of both the solid phase enzyme immunoassay and nucleic acid hybridization assay, the solid surface may be separated from the liquid reagent containing the chromogenic material subsequent to the contacting of the solid surface and the liquid reagent. Thus, the solid surface may be analyzed for the presence of the second color state of the chromogenic material while the solid surface is separate from the liquid reagent. This has the advantage of easier handling of the solid surface such as, for example, when the solid surface is located at the tip of a dip-stick, comparing the color on the dip-stick to a color chart or inserting the dip-stick into an optical reader. When the solid surface is particulate, the color state of the particles may be read while the particles are concentrated on the surface of a microfiltration membrane. Thus, the solid surface may comprise a membrane for use, for example, in a dip-stick format or a plurality of water insoluble particles for use, for example, in an automated particle-microfilter format. The membrane may be constructed of various papers including cellulose, carboxymethylated cellulose, DEAE cellulose, cellulose phosphate, cellulose sulfate, nitrocellulose, carboxymethylated nitrocellulose, DEAE nitrocellulose, nitrocellulose phosphate, nitrocellulose sulfate and cellulose acetate/cellulose nitrate. The membrane may also be constructed from activated hydrophilic alcohol-insoluble polyamide membrane available commercially

**0231010**

in pore sizes ranging from about 0.2-10.0u as Biodyne
or Carboxydyne Immuno Affinity Membranes from Pall Ultrafine
Filtration Corporation, Biotechnology Division, Glen
Cove, New York 11542. The particles may be beads such
as polystyrene.

The enzyme may be selected from horseradish
peroxidase ("HRP"), alkaline phosphatase and
beta-galactosidase. The liquid reagents containing the
chromogenic material may also contain a redox reagent
which reacts with the enzyme to cause the chromogenic
material to change from the first color state to a second
color state. The change in color states may refer to
a change in intensity of the same color, such as changing
from one intensity of a particular blue wavelength to
a greater or lesser intensity of the same blue wavelength.
The change in color state may alternatively mean a change
from one color to another such as, for example, a change
from clear to blue or a change from yellow to blue.
The chromogenic material may be a material which contains
a benzidine moiety, including 3,3'-dichlorobenzidine,
3,3',5,5'-tetramethylbenzidine, dianisidine, ortho-toluidine,
3,3'-diaminobenzidine, benzidine, 3-amino-9-ethylcarbazole,
and 4-chloro-1-naphthol.

The foregoing invention is illustrated by the
use of polyamide membrane or carboxymethylated cellulose
paper in combination with 3,3',5,5'-tetramethylbenzidine
("TMB"). Because of the presence of activated and functional
groups, the membrane and paper, respectively, are capable
of ionic bonding as well as other physico-chemical and
biochemical interactions including, for example, hydrophilic, hydrophobic and immunologic interactions. The enzyme-labeled membrane and paper are capable of binding with TMB. Enzyme-labeled DEAE cellulose is capable of similar binding with its diethylaminoethyl residue functional group and will bind with other chromogenic materials.

The present invention further provides for an article of manufacture, i.e. a dip-stick, comprising a porous membrane constructed from the above discussed membrane or papers. The membrane has opposed and parallel first and second planar surfaces, the distance between the first and second surfaces being no greater than 0.5 millimeters. The first surface has a first reagent access zone and a first binding zone which are substantially contiguous. The second surface may have a second reagent access zone substantially opposed to the first reagent access zone. The dip-stick is further constructed of carrier means fixedly coupled to the membrane at the first binding zone for rigidly supporting the first and second surfaces such that they remained opposed, parallel and planar when immersed in a liquid or otherwise wetted. The portion of a surface of the membrane designated as the reagent access zone is suitable for contacting reagents when the dip-stick is immersed. The reagent access zone is also the surface area of the membrane which reagents may contact when such reagents are dropped onto the dip-stick.

As an alternative, the second surface may further have a second binding zone which is contiguous to the second reagent access zone and which is substantially opposed to the first binding zone The carrier may be also fixedly coupled to the membrane at the second binding zone. The carrier may comprise opposed and parallel first and second leaflets. The membrane may be sandwiched between the first and second leaflets with the first binding zone being coupled to the first leaflet and the second binding zone being coupled to the second leaflet. The first and second leaflets may have first and second apertures respectively, with the first and second apertures being opposed respectively to the first and second reagent access zones so that reagents may contact such access zones without obstruction by the leaflets. The apertures may be completely circumscribed by the first and second leaflets respectively, thereby forming apertures having a cross section, for example, of a square, rectangle, circle, or other enclosed geometric shape. When such dip-stick is prepared for use in the above-described solid phase enzyme immunoassay or nucleic acid hybridization assay, the membrane will have attached to it an immunoreactant useful in such immunoassay or a single-stranded first fragment of nucleic acid of at least 15 base pairs useful in an enzyme nucleic acid hybridization assay.

The invention still further provides for a method of preparing a stabilized chromogenic reagent suitable for use in an enzyme immunoassay or an enzyme nucleic acid hybridization assay as well as other uses. The method provides for solubilizing a chromogenic material and a complementary redox agent. The chromogenic material may contain a benzidine moiety, the material being capable of changing from a first color state to a second color

- 10 -

state in correlation to an enzymatic reaction with a redox reagent. The complementary redox reagent is used in an amount effective to retain the chromogenic material in its first color state during normal handling and storage conditions by inhibiting redox reaction causing the chromogenic material during said normal handling and storage conditions to change to the second color state. The amount should also be effective to allow the chromogenic material to change from its first color state to the second color state as a result of redox reaction normally present for test positives in an enzyme immunoassay or an enzyme nucleic acid hybridization assay.

The chromogenic material containing the benzidine moiety may be selected from 3,3'-dichlorobenzidine, TMB, dianisidine, ortho-tolidine, 3,3'-diaminobenzidine, benzidine, 3-amino-9-ethylcarbazole and 4-chloro-1-naphthol. Where the chromogenic material is TMB, the complementary redox reagent may be formic acid. The chromogenic material and complementary redox reagents may be solubilized by mixing the two together along with a solubilizing agent such as dimethylsulfoxide ("DMSO"), dimethylformamide ("DMF"), methanol, ethanol, or other alcohol. A composition of matter may be formed according to the foregoing method.

Brief Description of the Figures

FIG. 1 shows a top view of a preferred embodiment dip-stick.

FIG. 2 shows a side view of the preferred embodiment dip-stick.

FIG. 3 shows an alternative embodiment dip-stick.

FIG. 4 shows a second alternative embodiment dip-stick.

FIG. 5 shows a third alternative embodiment dip-stick.

## DESCRIPTION OF THE PREFERRED EMBODIMENT

The present invention is illustrated by a system of dip-stick screening tests useful for the physician office. Allergy is one of the most common diseases and it is estimated that in the United States 35 million people suffer from this disease. An estimated 38 million patient visits per year are attributed to allergy and only 15% of that number of patient visits involve the practicing allergist. When care is administered by a trained allergist, immunotherapy can provide relief for the appropriately-selected patient. Thus it is of great value for primary care physicians to identify allergy sufferers for referrals to an allergist. When the primary care physician is confronted with a patient having rhinitis symptoms suggestive of allergies, the primary care physician will now have the option of a quick confirmation of a diagnosis. In addition to providing evidence for the presence of allergic sensitivity, the physician will be able to identify which group of antigens are involved.

The system of dip-stick screening tests chosen to illustrate the present invention will screen for trees, grasses, leaves, molds, foods, and a combination of several domestic allergens such as cats, dogs, and house dust. The tests have been designed to be rapid and simple to perform. The dip-stick can be evaluated qualitatively (yes/no) or quantitatively with a reflectometer, preferably a laser diode reflectometer.

While the present invention is illustrated by a system of dipstick screening tests for allergen-specific IgE, the invention could have been illustrated as well for a detection system for IgG, as well as other analytes detectable by immunoassay or detectable by nucleic acid hybridization assay.

1. Preparation of Membrane.

Biodyne Immuno Affinity Membrane, available commercially as #BIA 0308C5 Immunodyne membrane from Pall Ultrafine Filtration Corporation, Biotechnology Division, Glen Cove, N.Y. 11542, may be used as the membrane. This membrane, referred to herein as the polyamide membrane, is an activated hydrophilic alcohol-insoluble polyamide membrane manufactured according to the processes described in U.S. Patent No. 4,340,479, which patent is incorporated herein in its entirety by reference thereto. The membrane has a 3.0 u pore size. The polyamide membrane was chosen because of its ability to bind relatively large quantities of allergen for use in a solid phase sandwich assay for IgE. The polyamide membrane was also chosen for its good washing properties when used in conjunction with the following described dip-stick design.

Allergens may be bound individually or in mixtures of the following:

1. Weeds: short ragweed, lambs quarters, English plantain, mugwert, and Russian thistle;

2. Grasses: perennial rye, meadowfescue, timothy orchard and June (Kentucky blue);

3. Trees: oak, elm, maple and cottonwood;

4.  Molds: <u>Aspergillus fumigatus</u>, <u>Alternaria</u>
    <u>tenuis</u> and <u>Cladosporium herbarum</u>;

5.  Foods: milk products, grain products,
    vegetable products, fruit products and
    meat products; and

6.  Domestics: cat and dog epithelium, house
    dust and <u>D. farinae</u>.

Mixtures may be made from the above listed allergen preparations in ratios commonly found in industry. The above allergen preparations are available commercially.

The membranes are coated with the allergen according to the following procedure. Glycerinated or aqueous allergen extracts or mixtures purchased from commercial sources are treated in various known ways to remove extraneous material. The membrane is cut into desired sizes for coating with allergen. The membrane is placed into a positive pressure, flow-through filtration device. Allergen preparation at pH 5.0 is then placed into the filtration device and forced to flow through the membrane by positive pressure. The filtrate is then collected and recirculated through the membrane nine more times. A solution of 1% w/v human serum albumin ("HSA"), pH 9.0, is then added to the filtration device and forced through the membrane by positive pressure. Alternatively, specific allergen RAST class zero pooled human serum may be used instead of 1% HSA. A solution of 10% v/v $H_2O_2$ is added to the filtration device and passed through the membrane by gravity flow. The membrane is then air-dried and stored at $4^\circ$ C until used in manufacturing the dip-stick. When intended for use in

- 14 -

manufacturing the dip-stick, the membrane should be allowed to return to room temperature prior to such manufacture.

Alternatively, the membrane is wicked with a solution of .01 M sodium phosphate buffer, .15 M NaCl, pH 8.0. The membrane is then oriented in front of a macro-dropule spraying device and the allergen preparation is sprayed onto the wicked membrane. The membrane is then sprayed with a solution of 1% w/v HSA, pH 9.0. Alternatively, specific allergen RAST class zero pooled human serum may be used instead of 1% HSA. The membrane is then wicked with a solution of 10% v/v $H_2O_2$. The membrane is then air-dried and stored as above.

As a further alternative, immediately prior to use, the pH of the allergen preparation is adjusted to pH 5.0 with HCl. The allergen preparation is poured onto the membrane so as to completely soak the membrane. The membrane and allergen preparation are then allowed to bind 2-12 hours with gentle agitation at $4^{\circ}$ C. After binding, the membrane is rinsed three times with phosphate buffered saline, pH 7.2 ("PBS") and the PBS is then removed. The membrane is then treated with 1 mg/ml HSA in PBS buffer for at least 2-3 hours at $23^{\circ}$ C. Alternatively, the membrane may be treated with specific allergen RAST class zero pooled human serum instead of HSA. The membrane is then rinsed three times with distilled water. The membrane is then air-dried and stored at $4^{\circ}$ C until used in the manufacture of the dip-stick. When intended for use in manufacturing the dip-stick, the membrane should be allowed to return to room temperature prior to such manufacture.

2. Preparation of Dip-Stick

The dip-stick is constructed from polystyrene and the allergen-coated membrane. Double sided tape using an acrylic adhesive on a polyester carrier is applied to one side of a large sheet of polystyrene by removing the backing from one side of such tape and tightly pressing it against the sheet of polystyrene. This procedure is repeated for a second sheet of polystyrene. One sheet is then placed on top of the other with the tape from each membrane opposed to one another. At this point, the two sheets of tape will not stick together because they are separated by each of their backings. Register holes are then punched in the sheets of polystyrene to assure proper registration throughout the following procedures.

While the two sheets of polystyrene are registered, rows of apertures are punched through the two sheets of polystyrene. The top polystyrene sheet is then removed and the remaining backing on each sheet of tape is then removed. Narrow strips of the coated membrane prepared according to the above procedure is then placed onto one of the sheets of tape so as to be positioned over the row of apertures. The second sheet of polystyrene is then placed onto the coated membrane strip so as to form a sandwich of coated strips between the tape of each sheet of polysytrene. The registration holes will guarantee that the apertures of each sheet will be aligned in opposition to one another. The sandwich is then pressed together and individual dip-sticks are cut out with a 25 ton shear press.

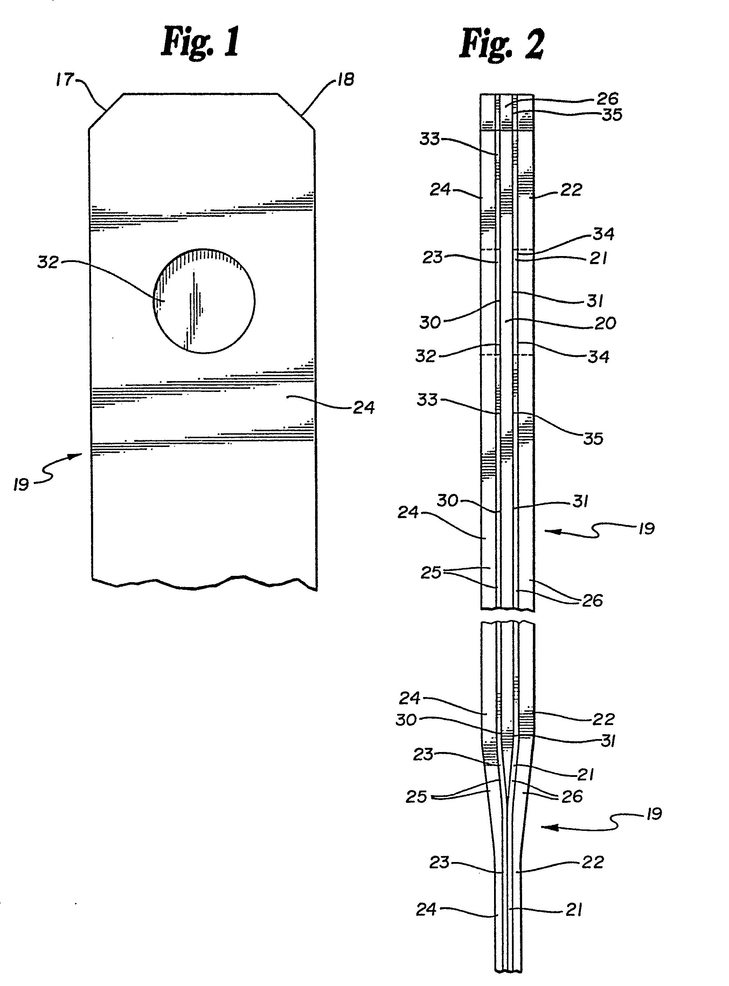

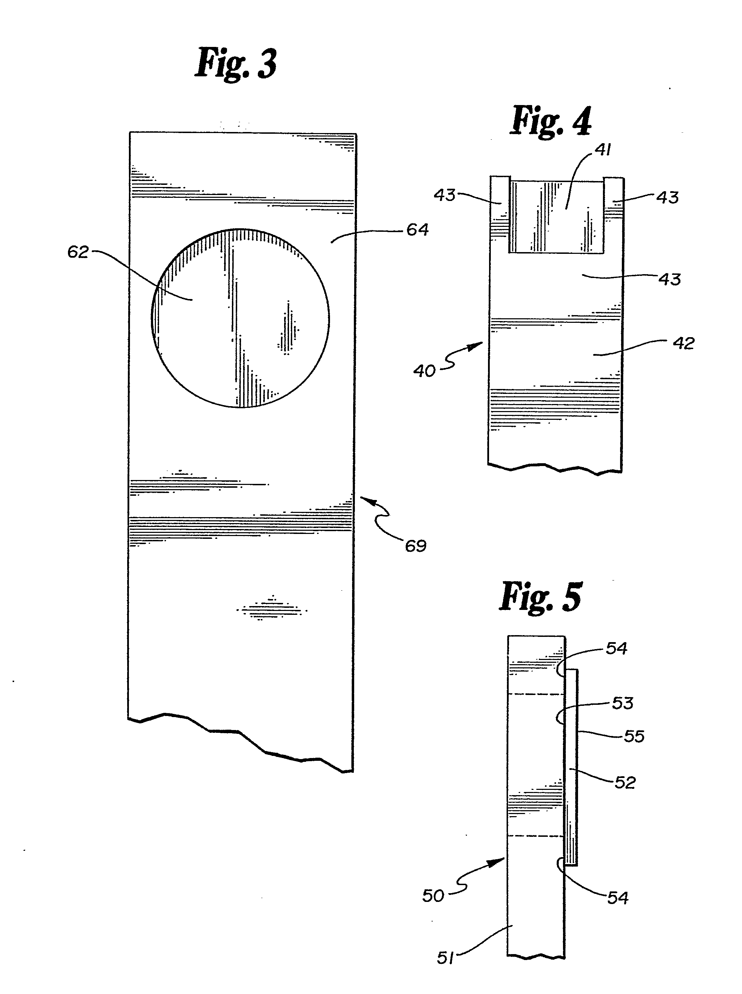

The resulting dip-sticks are illustrated in Figs. 1 and 2. Dip-stick 19 is constructed from porous membrane 20 which in the preferred embodiment is prepared according to the above procedures. Membrane 20 has first surface 30 and second surface 31 which are each planar and together are opposed and parallel. The distance between first surface 30 and second surface 31 defines the thickness of membrane 20, which is no greater than 0.5 millimeters in the preferred embodiment. First surface 30 has first reagent access zone 32 and first binding zone 33. Second surface 31 has second reagent access zone 34 and second binding zone 35. First reagent access zone 32 is substantially opposed to second reagent access zone 34 and first binding zone 33 is substantially opposed to second binding zone 35.

In constructing dip-stick 19 according to the above method, an aperture of about 3.0 mm may be punched through the registered sandwich consisting of polystyrene sheet 24, double-sided tape 23, double-sided tape 21 and polystyrene sheet 22. When membrane 20 is sandwiched between the foregoing, first reagent access zone 32 shows through the aperture in the top view of dip-stick 19 as shown in FIG. 1. The remaining portions of first surface 30 is first binding zone 33. First binding zone 33 is obscured wholly by polystyrene sheet 24 in FIG. 1. Because the aperture cuts through polystyrene sheet 24 and double-sided tape 23, the aperture defines first reagent access zone 32. First binding zone 33 is substantially contiguous to first reagent access zone 32. Second binding zone 35 is similarly substantially contiguous to second reagent access zone 34 for the same reason. Because the aperture was cut through polystyrene sheet 22 and double-sided tape 21 while they were in registration with polystyrene sheet 24 and double-sided tape 23, the respective binding zones are substantially opposed and the respective reagent access zones are similarly substantially opposed.

The two polystyrene sheets 22 and 24 in combination with the two double-sided tape 21 and 23 form a carrier which is coupled to membrane 20 at first binding zone 33 and second binding zone 35. The portion of polystyrene sheet 24 and double-sided tape 23 which are opposed to membrane 20 constitutes first leaflet 25. The portion of polystyrene sheet 22 and double-sided tape 21 which is also opposed to membrane 20 constitutes second leaflet 26. Membrane 20 is thus sandwiched between first and second leaflets 25 and 26 with first binding zone 33 coupled to first leaflet 25 and second binding zone 34 coupled to second leaflet 26.

The dip-stick is beveled at a 45° angle on each corner of the end of the dip-stick at 17 and 18.

Because the shape of the aperture was chosen to be a circle, the aperture punched into the first leaflet is completely circumscribed by the first leaflet. Similarly, the aperture which is punched into the second leaflet and which is directly opposed to the first leaflet is completely circumscribed by the second leaflet.

As an alternative embodiment of the dip-stick, FIG. 3 shows dip-stick 69 where the aperture of the reagent access zones 62 is increased to a diameter of 5.0 mm and the end of the dip-stick is not beveled as in FIG. 1.

As a second alternative embodiment of the dip-stick, FIG. 4 shows dip-stick 40 where the aperture is not completely circumscribed by the relevant leaflet. The top view of dip-stick 40 shows the portion of the membrane which constitutes the first reagent access zone 41. The first binding zone is obscured by polystyrene sheet 42. The top view in FIG. 4 shows first leaflet 43 which is bound to the membrane. A bottom view would look identical showing a second leaflet. Dip-stick 40 in FIG. 4 is constructed similarly to dip-stick 19 in that membrane 20 is sandwiched between two polystyrene sheets and two sheets of double-sided tape.

As a third alternative embodiment of the dip-stick, FIG. 5 shows dip-stick 50. Membrane 52 has first reagent access zone 53 and first binding zone 54. Membrane 52 further has second reagent access zone 55. Dip-stick 50 shown in FIG. 5 is constructed from a single polystyrene sheet having an aperture opposed to a membrane bound on one side of the polystyrene sheet. The membrane may be bound to the polystyrene by use of a single sheet of double-sided tape or by other means. As in the case of the preferred and other alternative embodiments, the first reagent access zone and first binding zone are substantially contiguous and the second reagent access zone is substantially opposed to the first reagent access zone. The carrier which is made up of polystyrene sheet 51 is fixedly coupled to membrane 52 at first binding zone 54.

As further alternative embodiments of the dip-stick, the aperture may be a square, oval or other design or geometric figure and the reagent access zone may or may not be completely circumscribed by the binding zone.

The above dip-stick design has in common a threefold advantage. First, by exposing both sides of the coated membrane to a reagent, the assay sensitivity is significantly increased because a significant portion of the immunoreaction or nucleic acid hybridization reaction occurs in the interstices of the membrane and not just on the surface alone. By having a design where both surfaces of the membrane are exposed to the reagent, there will be diffusion of the reagent into the membrane from both sides rather than simply from a single side. Second, by rigidly holding a thin membrane in a planar configuration, a broad surface is exposed which is of use in washing the membrane while performing such assays. The planar configuration allows the use of pulsing machines which can easily force washing fluid through the thin planar membrane. Third, the dip-stick and reagent access area may be sized to fit commercially available optical readers. This advantage presupposes that the color change is present on and in the membrane rather than in the chromogen substrate solution.

3. Preparation of Chromogen.

A stabilized TMB solution which is stable in liquid phase and retains the chromogenic characteristics of freshly-prepared TMB in a peroxide/peroxidase reaction may be prepared as follows. The stabilized TMB solution is stable in its concentrated form and requires dilution in buffer containing peroxide immediately prior to its use in an assay.

50 mg TMB, available commercially from Sigma, St. Louis, Mo., is mixed with 5.0 ml 0.1 N HCl to suspend the TMB. 0.1 ml 90% (vol.) formic acid/water are mixed with the foregoing until the TMB forms a fine suspension. 5.0 ml dimethylsulfoxide ("DMSO") is added and mixed until the solution becomes clear. The clear solution is stored in an opaque container.

The foregoing stabilized TMB solution is stable at $37^{\circ}$ C at least one week and at $23^{\circ}$ C at least one month. That is, at these temperature ranges and for these periods of time the stabilized TMB solution will yield a colored product at a rate equivalent to that of using a freshly-prepared TMB solution. The stabilized TMB solution will undergo a slight color change when exposed to light. The solution should be stored in an opaque container. However, the reaction is reversible. The stabilized TMB solution readily mixes with aqueous buffers and does not separate or precipitate. The colored TMB, a product of an enzyme redox reaction, will adhere to certain types of active membrane surfaces.

For the purposes of the following assay protocol, the stabilized TMB solution will be diluted to about 0.2 mg/ml final concentration in a 0.1 M citrate/acetate buffer at pH 6.0 containing hydrogen peroxide. The diluted product is stable for about two hours at $21^{\circ}$ C.

4. Preparation of Ancillary Reagents.

Anti-human IgE antibody, available commercially, of at least 50 ng/ml, preferably about 300 ng/ml, in
0.1 M TRIS buffer, pH 7.4, with 10% v/v fetal bovine
serum ("FBS"), 0.1 M NaCl, 20 mM $CaCl_2$, 10 ug/ml Gentamicin
sulphate, 0.1 g/l Thimerasol and 0.1% Triton X-100.

A wash solution concentrate may be prepared
using 0.05% (vol.) tween-20 in normal saline.  30 ml
of wash solution concentrate is diluted with one liter
of distilled or deionized water for washing procedures.
Substrate buffer may be prepared by adding 0.1 M citric
acid to 0.1 M sodium acetate to bring the pH to 6.0.
Hydrogen peroxide is then added to bring its concentration
to about 0.0002% (vol.).  Control serum containing known
amounts of IgE may be prepared according to known
procedures.  The above reagents should be stored at 2-8°
C until used.

5.  Assay Protocol.

Prior to performing the assay, the dip-stick
prepared according to the above procedures should be
allowed to equilibrate to room temperature.

100 ul of patient serum is added to a well.
The dip-stick is then placed into the patient's serum
so that the membrane is completely immersed.  The coated
membrane is allowed to incubate with the patient's serum
for 30-45 minutes at room temperature.  Following incubation,
the membrane is washed by aiming a forceful stream of
wash solution directly on the membrane for five to seven
seconds on each side, making sure that the entire surface
of the membrane has been rinsed.  The dip-stick is then
inserted into a well containing 100 ul HRP-labeled anti-human
IgE conjugate and allowed to incubate at room temperature for 30-45 minutes. Five minutes before the end of the second incubation period, 100 ul of TMB solution is added to 5 ml of substrate solution. 250 ul of the resulting solution is then added into an empty well. At the end of the second incubation period, the dip-stick should then be removed from the well containing HRP-labeled conjugate and washed as set forth above. The dip-stick is then placed in a well containing the above-described TMB/substrate solution and allowed to react at room temperature for 15-25 minutes. The dip-stick is then removed from the well and the membrane is briefly rinsed (2-3 seconds per side) with the above wash solution or tap water. Following rinsing, the membrane may be gently blotted to remove water and read for change of color state within one minute. Alternatively, the dip-stick may stand in distilled or deionized water for up to 30 minutes without loss of color. Serum controls may be used to insure the validity of results obtained.

The dip-stick is then inserted into a laser diode reflectometer and the degree of chromogen development is determined.

### DESCRIPTION OF AN ALTERNATIVE EMBODIMENT

1. Preparation of Membrane.

Carboxymethylated cellulose, available commercially as membrane #NA49 from Schleicher & Schuell, Inc., Keene, N.H. 03431, may be used as the membrane. This paper is composed of carboxymethylated cellulose fibers (0.45 um pore size and approximately 0.0076 cm thickness) with the carboxylic acid as the functional group. This paper was chosen because of its ability to bind relatively large quantities of allergen for use in a solid phase sandwich assay for IgE. The paper was also chosen for its good washing properties when used in conjunction with the following described dip-stick design.

Allergens are bound individually or in mixtures similar to the following mixtures:

1. Weed mix: short ragweed, lambs quarters, English plantain, mugwert, and Russian thistle;

2. Grass mix: perennial rye, meadowfescue, timothy orchard and June (Kentucky blue);

3. Tree mix: oak, elm, maple and cottonwood;

4. Mold mix: Aspergillus fumigatus, Alternaria tenuis and Cladosporium herbarum; and

5. Domestic mix: cat and dog epithelium, house dust and D. farinae.

The above five mixtures are made from the above-listed allergen extracts in ratios commonly found in industry. The above allergen extracts are available commercially.

The membranes are coated with the allergen mixtures according to the following procedure. Glycerinated or aqueous allergen extracts or mixes purchased from commercial sources are treated in various known ways to remove extraneous material. The carboxymethylated cellulose paper is cut into desired sizes for coating with allergen. The paper is soaked in 0.0125 M sodium phosphate pH 5.0 for 15 minutes. It is important that the paper be entirely covered. The above buff is removed from the paper just prior to adding prepared extract.

Immediately prior to use, the pH of the extract
is adjusted to 8.0 with NaOH. EDAC
(1-ethyl-3-(3-dimethylamino-propyl) carbodiimide) is
added. The extract and EDAC are mixed gently and allowed
to stand for 15 minutes. This mixture is poured onto
the paper so as to completely soak the paper. The paper
and extract are then allowed to bind overnight with gentle
agitation at $4^\circ$ C. After overnight binding, the paper
is rinsed three times with phosphate-buffered saline,
pH 7.2 ("PBS") and the PBS is then removed. The paper
is then treated with 1 mg/ml HSA in PBS buffer for at
least 2-3 hours at $23^\circ$ C. The paper is then rinsed three
times by 10% (vol.) glycerine/water solution. After
the paper is drained of glycerine, it is spread out on
blotter paper for one minute and then sandwiched between
two pieces of blotter paper and immediately frozen at
$-40^\circ$ C or lower. The sandwich is then lyophilized and
stored at $4^\circ$ C until used in the manufacture of the
dip-stick. When intended for use in manufacturing the
dip-stick, the sandwich should be allowed to return to
room temperature prior to such manufacture.

2. Preparation of Dip-Stick

The dip-stick is constructed from polystyrene
and the allergen-coated membrane by the same process
and in the same embodiments disclosed above in the description of the preferred embodiment, which disclosure is
incorporated herein in its entirety by reference thereto.

3. Preparation of Chromogen.

A stabilized TMB solution which is stable in
liquid phase and retains the chromogenic characteristics of freshly-prepared TMB in a peroxide/peroxidase reaction may be prepared as follows. The stabilized TMB solution is stable in its concentrated form and requires dilution in buffer containing peroxide immediately prior to its use in an assay.

100 mg TMB, available commercially from Sigma, St. Louis, Mo. is mixed with 5.0 ml 0.1 N HCl to suspend the TMB. 0.1 ml 90% (vol.) formic acid/water are mixed with the foregoing until the TMB forms a fine suspension. 5.0 ml dimethylsulfoxide (DMSO) is added and mixed until the solution becomes clear. The clear solution is stored in an opaque container.

The foregoing stabilized TMB solution is stable at 37° C at least one week and at 23° C at least one month. That is, at these temperature ranges and for these periods of time the stabilized TMB solution will yield a colored product at a rate equivalent to that of using a freshly-prepared TMB solution. The stabilized TMB solution will undergo a slight color change when exposed to light. The solution should be stored in an opaque container. However, the reaction is reversible. The stabilized TMB solution readily mixes with aqueous buffers and does not separate or precipitate. The colored TMB, a product of an enzyme redox reaction, will adhere to certain types of active membrane surfaces.

For the purposes of the following assay protocol, the stabilized TMB solution will be diluted to about 0.2 mg/ml final concentration in a 0.1 M citrate/acetate buffer at pH 6.0 containing hydrogen peroxide. The diluted product is stable for about two hours at 21° C.

- 26 -

4. Preparation of Ancillary Reagents.

Anti-human IgE antibody, available commercially, is labeled with horseradish peroxidase ("HRP"), available commercially, according to known procedures or HRP-labeled anti-human IgE antibodies is purchased. HRP-labeled anti-human IgE antibody is diluted to a final concentration of at least 50 ng/ml, preferably about 450 ng/ml, in 0.1 M TRIS buffer, pH 8.0, containing 2 mg HSA per ml.

A wash solution concentrate may be prepared using 0.05% (vol.) tween-20 in normal saline. 30 ml of wash solution concentrate is diluted with one liter of distilled or deionized water for washing procedures. Substrate buffer may be prepared by adding 0.1 M citric acid to 0.1 M sodium acetate to bring the pH to 6.0. Hydrogen peroxide is then added to bring its concentration to about 0.0005% (vol.). Control serum containing known amounts of IgE may be prepared according to known procedures. The above reagents should be stored at 2-8° C until used.

5. Assay Protocol.

Prior to performing the assay, the dip-stick prepared according to the above procedures should be allowed to equilibriate to room temperature. Within two hours prior to performing the assay, three drops of the chromogen concentrate prepared according to the above procedures are added to 5 ml of substrate buffer and mixed gently. The resulting solution should be kept at room temperature until used.

A small amount of patient serum is added to a well. If an optical reader or reflectometer is to be used, a dry, unreacted dip-stick should be inserted into the reader in order to calibrate the reader. The dip-stick is then be placed into the patient's serum so that the membrane is completely immersed. The coated membrane is allowed to incubate with the patient's serum for 15 minutes at room temperature. After 15 minutes, the membrane is washed by aiming a forceful stream of wash solution directly on the membrane for five to seven seconds on each side, making sure that the entire surface of the membrane has been rinsed. The dip-stick is then inserted into a well containing HRP-labeled anti-human IgE conjugate and allowed to incubate at room temperature for 15 minutes. The dip-stick should then be removed and washed as set forth above. The dip-stick is then placed in a well containing the above-described TMB solution and allowed to react at room temperature for five minutes. The dip-stick is then removed from the well and the membrane briefly rinsed (2-3 seconds per side) with the above wash solution or tap water. Following rinsing, the membrane may be gently blotted to remove water and read for change of color state within one minute. Alternatively, the dip-stick may stand in distilled or deionized water for up to 30 minutes without loss of color. Serum controls may be used to insure the validity of results obtained.

From the foregoing, it will be obvious to those skilled in the art that various modifications in the above described methods can be made without departing from the spirit and scope of the invention. Accordingly, the invention may be embodied in other specific forms without departing from the spirit or esential characteristics thereof. Present embodiments, therefore, are to be considered in all respects as illustrative and not restrictive, the scope of the invention being indicated by the appended claims rather than by the foregoing description, and all changes which come within the meaning and range of equivalency of the claims are therefore intended to be embraced therein.

CLAIMS

1. A solid phase enzyme immunoassay wherein the presence of an analyte in a liquid sample is determined by contacting (i) a solid surface having attached thereto an immunoreactant specific for said analyte with (ii) said liquid sample and with (iii) an enzyme labeled material selected from the group consisting of material which selectively binds with said immunoreactant and material which selectively binds with said analyte, said presence of said analyte in said sample correlating to the amount of said enzyme labeled material bound to said solid surface resulting from said contacting; which comprises:

    (a) contacting said solid surface, having bound thereto said enzyme labeled material, with a liquid reagent containing a chromogenic material, said chromogenic material being capable of changing from a first color state to a second color state in correlation to the amount of said enzyme labeled material bound to said solid surface, and said chromogenic material being capable, when in said second color state, of substantially binding to said solid surface; and

    (b) analyzing the solid surface for the presence of the second color state.

2. A solid phase nucleic acid hybridization assay wherein the presence of single-stranded analyte nucleic acid in a liquid sample is determined by contacting (i) a solid surface having attached thereto a single-stranded first fragment of nucleic acid having a nucleotide sequence of at least 15 base pairs and being capable of hybridizing with said analyte nucleic acid with (ii) said liquid sample and with (iii) an enzyme labeled single-stranded second fragment of nucleic having a nucleotide sequence of at least 15 base pairs, said second fragment of nucleic acid being selected from the group consisting of second fragment of nucleic acid which selectively hybridizes with said analyte nucleic acid and second fragment of nucleic acid which selectively hybridizes with said first fragment of nucleic acid, said presence of analyte nucleic acid in said liquid sample correlating to the amount of enzyme labeled second fragment of nucleic acid bound to said solid surface as a result of said contacting;

which comprises:

(a) contacting said solid surface, having bound thereto said enzyme labeled material, with a liquid reagent containing a chromogenic material, said chromogenic material being capable of changing from a first color state to a second color state in correlation to the amount of said enzyme labeled material bound to said solid surface, and said chromogenic material being capable, when in said second color state, of substantially binding to said solid surface; and

b) analyzing the solid surface for the presence of the second color state.

3. The solid phase assay of claim 1 or 2, wherein the solid surface is separated from said liquid reagent subsequent to the contacting of step a) and prior to the analyzing of step b).

4. The solid phase assay of any of the claims 1 to 3, wherein said solid surface comprises a membrane.

5. The solid phase assay of claim 4, wherein said membrane comprises a material selected from the group consisting of cellulose, carboxymethylated cellulose, DEAE cellulose, cellulose phosphate, cellulose sulfate, nitrocellulose, carboxymethylated nitrocellulose, DEAE nitrocellulose, nitrocellulose phosphate, nitrocellulose sulfate, cellulose acetate/cellulose nitrate and a polyamide membrane.

6. The solid phase assay of any of the claims 1 to 3, wherein said solid surface is selected from the group consisting of a membrane contained in a dip-stick, a membrane contained in a microtiter well, and a treated or coated surface of a test tube.

7. The solid phase assay of any of the claims 1 to 3, wherein said solid surface comprises a plurality of water insoluble particles.

8. The solid phase assay of any of the claims 1 to 7, wherein the enzyme is selected from the group consisting of horseradish peroxidase, alkaline phosphatase and beta-galactosidase.

9. The solid phase assay of any of the claims 1 to 8, wherein the liquid reagent further contains a redox reagent which reacts with said enzyme to cause said chromogenic material to change from said first color state to said second color state.

10. The solid phase assay of claim 9, wherein the redox reagent comprises hydrogen peroxide.

11. The solid phase assay of any of the claims 1 to 10, wherein the chromogenic material contains a benzidine moiety.

12. The solid phase assay of claim 11, wherein the chromogenic material containing a benzidine moiety is selected from the group consisting of 3,3'-dichlorobenzidine, 3,3',5,5'-tetramethylbenzidine,

0231010

dianisidine, <u>ortho</u>-toluidine, 3,3'-diaminobenzidine, benzidine, 3-amino-9-ethylcarbozole and 4-chloro-1-naphthol.

13. An article of manufacture comprising:

a porous membrane comprising

a material selected from the group consisting of cellulose, carboxymethylated cellulose, DEAE cellulose, cellulose phosphate, cellulose sulfate, nitrocellulose, carboxymethylated nitrocellulose, DEAE nitrocellulose, nitrocellulose phosphate, nitrocellulose sulfate, cellulose acetate/cellulose nitrate and polyamide membrane, said membrane having opposed and parallel first and second planar surfaces, the distance between said first and second surfaces being no greater than 0.5 millimeters; said first surface having a first reagent access zone and a first binding zone which are substantially contiguous, said second surface having a second reagent access zone substantially opposed to said first reagent access zone; and

carrier means fixedly coupled to said membrane at said first binding zone for rigidly supporting said first and second surfaces such that said first and second surfaces remain opposed, parallel and planar when immersed in a liquid or otherwise wetted.

0231010

14. The article of manufacture of claim 13, wherein said second surface further has a second binding zone which is contiguous to said second reagent access zone and which is substantially opposed to said first binding zone, and said carrier is further fixedly coupled to said membrane at said second binding zone.

15. The article of manufacture of claim 13 or 14, wherein the carrier comprises opposed and parallel first and second leaflets, said membrane being sandwiched between said first and second leaflets with said first binding zone being coupled to said first leaflet and said second binding zone being coupled to said second leaflet.

16. The article of manufacture of claim 15, wherein said first and second leaflets have first and second apertures respectively, said first and second apertures being opposed respectively to said first and second reagent access zones.

17. The article of manufacture of claim 16, wherein said first and second apertures are respectively completely circumscribed by said first and second leaflets.

18. The article of manufacture of any of the claims 13 to 17, wherein a portion of said membrane has attached an immunoreactant useful in an enzyme immunoassay or a single-stranded fragment of nucleic acid of at least 15 base pairs useful in an enzyme nucleic acid hybridization assay.

19. A method of preparing a stabilized chromogenic reagent suitable for use in an enzyme immunoassay or enzyme nucleic acid hybridization assay, said method comprising solubilizing:

a chromogenic material containing a benzidine moiety, said chromogenic material being capable of changing from a first color state to a second color state in correlation to an enzyme's reaction with a redox reagent; and

a complementary redox agent in an amount (i) effective to retain the chromogenic material in its first color state during normal handling and storage conditions by inhibiting redox reaction causing said chromogenic material during said normal handling and storage conditions to change to said second color state, and (ii) effective to allow the chromogenic material to change from its first color state to the second color state as a result of redox reactions normally present for test positives in an enzyme immunoassay or an enzyme nucleic acid hybridization assay.

20.       The method of claim 19, wherein the chromogenic
material containing a benzidine moiety is selected from
the group consisting of 3,3'-dichlorobenzidine,
3,3',5,5'-tetramethylbenzidine, dianisidine, <u>ortho</u>-toluidine,
3,3'-diaminobenzidine, benzidine, 3-amino-9-ethylcarbazole
and 4-chloro-1-naphthol.

21.       The method of claim 19, wherein the complementary
redox agent is formic acid.

22.       The method of any of the claims 19 to 21, wherein
the chromogenic material and the complementary redox agent
are solubilized by mixing with said chromogenic material
and said complementary redox reagent with a solubilizing agent.

23.       The method of claim 22, wherein the solubilizing
agent is selected from the group consisting of
dimethylsulfoxide, dimethylformamide, methanol and ethanol.

24.       The method of claim 22 or 23, wherein the chromogenic
material is 3,3',5,5' - tetramethylbenzidine and the
complementary redox agent is formic acid.

25.     As a composition of matter, a solution comprising:

a chromogenic material containing a benzidine moiety, said chromogenic material being capable of changing from a first color state to a second color state in correlation to an enzyme's reaction with a redox reagent;

and

a complementary redox agent in an amount (i) effective to retain the chromogenic material in its first color state during normal handling and storage conditions by inhibiting redox reaction causing said chromogenic material during said normal handling and storage conditions to change to said second color state; and (ii) effective to allow the chromogenic material to change from its first color state to the second color state as a result of redox reactions normally present for test positives in an enzyme immunoassay or an enzyme nucleic acid hybridization assay.

26.     The composition of matter of claim 25, wherein
the chromogenic material containing a benzidine moiety
is selected from the group consisting of.
3,3'-dichlorobenzidine, 3,3',5,5'-tetramethylbenzidine,
dianisidine, ortho-toluidine, 3,3'-diaminobenzidine,
benzidine, 3-amino-9-ethylcarbazole and 4-chloro-1-naphthol.

0231010

27.  The composition of matter of claim 25 or 26, wherein the complementary redox agent is formic acid.

28.  The composition of matter of any of the claims 25 to 27, wherein the chromogenic material and the complementary redox reagent are solubilized by the presence of a solubilizing agent.

29.  The composition of matter of claim 28, wherein the solubilizing agent is selected from the group consisting of dimethylsulfoxide, dimethylformamide, methanol and ethanol.

30.  The composition of matter of claim 29, wherein chromogenic material is 3,3',5,5' – tetramethylbenzidine and the complementary redox reagent is formic acid.

Sl/JG

1/2

0231010

### Fig. 1

### Fig. 2

## Fig. 3

62
64
69

## Fig. 4

41
43
43
43
42
40

## Fig. 5

54
53
55
52
50
54
51